# EUROPEAN PATENT SPECIFICATION

(11) **EP 0 844 887 B1**
(45) Date of publication and mention of the grant of the patent: **23.12.2009**
(21) Application number: 96921468.3
(22) Date of filing: 05.06.1996
(51) Int. Cl.: C12N 15/86, A61K 48/00, C12N 5/00

(54) **AAV TRANSDUCTION OF MYOBLASTS**
DIE TRANSDUKTION VON MYOBLASTEN MITTELS VEKTOREN AUS ADENOASSOZIIERTEN VIREN
TRANSDUCTION DE MYOBLASTES PAR VECTEURS DE VIRUS ADENO-ASSOCIES

(30) Priority: 07.06.1995 US 487005
(43) Date of publication of application: 03.06.1998
(73) Proprietor: UNIVERSITY OF NORTH CAROLINA AT CHAPEL HILL, Chapel Hill, North Carolina 27599-4100 (US)
(72) Inventor: XIAO, Xiao, Chapel Hill, NC 27514 (US); SAMULSKI, Richard, J., Chapel Hill, NC 27514 (US)
(74) Representative: Vossius & Partner
(86) International application number: PCT/US1996/009879
(87) International publication number: WO 1996/040272

(56) References cited:
- WO-A-94/13788
- WO-A-97/12050
- WO-A-97/26337
- KOURTIS A P ET AL: "CARDIAC GENE THERAPY WITH ADENO-ASSOCIATED VIRUS AS A MEANS OF ARCHIEVING GRAFT-SPECIFIC IMMUNO-SUPPRESSION" MODERN PATHOLOGY,US,BALTIMORE, MD, vol. 8, no. 1, 1995, page 33A XP000673501 ISSN: 0893-3952
- KESSLER P D ET AL: "Gene delivery to skeletal muscle results in the sustained expression and systemic delivery of therapeutic proteins." JOURNAL OF INVESTIGATIVE MEDICINE, vol. 44, no. 3, 1996, page 279A XP000979013 Annual Meeting of the Association of American Physicians, the American Society for Clinical Investigation, and the American Federation for Clinical Research: Biomedicine '96, Medical Research from Bench to Bedside;Washington, D.C., USA; May 3-6, 1996 ISSN: 1081-5589
- BARTLETT R J ET AL: "A plasmid expression vector based on the adeno-associated virus (AAV) containing muscle-specific transcription elements for expression of therapeutic proteins." AMERICAN JOURNAL OF HUMAN GENETICS, vol. 57, no. 4 SUPPL., 1995, page A235 XP002158232 45th Annual Meeting of the American Society of Human Genetics;Minneapolis, Minnesota, USA; October 24-28, 1995 ISSN: 0002-9297
- PODSAKOFF G ET AL: "LONG-TERM IN VIVO GENE EXPRESSION IN MUSCLE USING AAV VECTORS" BLOOD,US,W.B. SAUNDERS, PHILADELPHIA, VA, vol. 86, no. 10, SUPPL. 01, 15 November 1995 (1995-11-15), page 1004A XP000673499 ISSN: 0006-4971
- SCIENCE, Volume 269, issued 25 August 1995, MARSHALL, "Gene Therapy's Growing Pains", pages 1050-1055, XP002926938.
- ADVANCED DRUG DELIVERY REVIEWS, Volume 12, issued 1993, XIAO et al., "Adeno-Associated Virus (AAV) Vectors for Gene Transfer", pages 201-215, XP000676928.
- CLINICAL RESEARCH, Volume 40, Number 02, issued 1992, MARCH et al., "The Adeno-Associated Virus as a Gene Transfer Vector for Human and Non-Human Vascular Smooth Muscle Cells", XP002940730.

## Description

### INTRODUCTION

### Technical Field

This invention is in the field of gene expression and is particularly directed to expression of gene products in the muscle of an animal.

### Background

Adeno-associated virus (AAV) vectors have been proposed and patented as vectors for expressing gene products in animals. See, for example, U.S. patent No. 5,193,941, issued 18 August 1992, WO 9413788 and 08/227,319, the last application arising from the laboratory of the present inventors. A number of patents and other publications describe numerous AAV vectors and their uses, the uses generally being related to expression of gene products either in vitro (usually tissue cultures) or in vivo (usually in the lungs or nasal mucosa, the normal sites of AAV infection, although U.S. application serial No. 08/227,319 relates to expression in the central nervous system).

Investigations in the laboratories of the present inventors have surprisingly discovered that AAV vectors can act as effective, long-term expression systems in the muscle tissue of animals after intramuscular injection. This discovery provides a new method of expressing desirable gene products and control elements in the muscle tissue of animals, including humans.

### SUMMARY OF THE INVENTION

Accordingly, it is an object of the invention to provide new uses for AAV vectors that have already been developed for other purposes.

It is a further object of the invention to provide new recombinant AAV vectors containing muscle tissue-directed gene expression systems.

These and other objects of the invention have been accomplished by providing a method of expressing a gene product in the muscle tissue of an animal, which comprises administering a recombinant AAV vector to the muscle tissue of the animal, wherein the vector comprises a non-AAV gene of interest ligated into an AAV vector.

### DESCRIPTION OF SPECIFIC EMBODIMENTS

The present invention is quite straightforward: prior to this invention recombinant AAV vectors were well known and were known to be able to transduce a number of cells and tissues, but had not been used or suggested for use in expressing gene products in the muscle tissue of animals. The invention therefore comprises administering to the muscle tissue of a target animal a recombinant AAV vector containing a gene whose expression is desired (along with the appropriate control elements, if desired or necessary in the normal manner for vectors). No new vectors are required, as previously known AAV vectors have been shown to work well for muscle tissue expression. Thus the invention is in part a discovery that no particular adaption of AAV vectors is required for muscle tissue expression, which is surprising in view of the strict requirements for AAV reproduction (i.e., presence of a helper virus) and the normal association of AAV with the lungs and nasal passages.

A number of scientific and patent publications describe the state of the art in the AAV vector field. Since no particular adaptations of prior art vectors are required for practice of the present invention, there is no need here to detail at great length the already well-known state of the art. However, the following publications are herein incorporated by reference, as are the patent and the patent applications (and their published equivalents) identified in the Introduction section of this specification, as these materials may be useful for those less experienced in the AAV field:
1. Samulski, R.J. et al. (1982) Proc. Natl. Acad. Sci. USA. 79:2077-2081 "Cloning of Adeno-Associated Virus into pBR322: Rescue of Intact Virus from Recombinant Plasmid in Human Cells"
2. Samulski, R.J. et al. (1983) Cell 33:135-143 "Rescue of Adeno-Associated Virus from Recombinant Plasmids: Gene Correction within the Terminal Repeats of AAV"
3. Laughlin et al. (1983) Gene 23:65-73 "Cloning of Infectious Adeno-Associated Virus Genomes in Bacterial Plasmids"
4. Hermanot, P.L. and Muzycka, N. (1984) Proc. Natl. Acad. Sci. USA. 81:6466-6470 "Use of Adeno-Associated Virus as a Mammalian DNA Cloning Vector: Transduction of Neomycin Resistance into Mammalian Tissue Culture Cells"
5. Senepathy, P. et al. (1984) J. Mol. Biol. 178, 179:1-20 "Replication of Adeno-Associated Virus DNA Complementation of Naturally Occurring rep- Mutants by a Wild-type Genome or an ori- Mutant and Correction of Terminal Palindrome Deletions"
6. Tratschin et al. (1984) J. Virol 51:611-619 "Genetic Analysis of Adeno-Associated Virus: Properties of Deletion Mutants Constructed In Vitro and Evidence for an Adeno-Associated Virus Replication Function"
7. Tratschin et al. (1984) Mol. Cell. Biol. 4:2072-2081 "A Human Parvovirus, Adeno-Associated Virus, as a Eukaryotic Vector: Transient Expression and Encapsidation of the Prokaryotic Gene for Chloramphenicol Acetyltransferase"
8. Miller et al. (1986) Somatic Cell and Molecular Genetics 12:175-183 "Factors Involved in Production of Helper Virus-Free Retrovirus Vectors"
9. Bosselman et al. (1987) Mol. Cell. Biol. 7:1797-1806 "Replication-Defective Chimeric Helper Proviruses and Factors Affecting Generation of Competent Virus: Expression of Moloney Murine Leukemia Virus Structural Genes via the Metallothionein Promoter"
10. Ohi et al. (1988) J. Cell. Biol. 107:304A "Construction and Characterization of Recombinant Adeno-Associated Virus Genome Containing β-globin cDNA"
11. McLaughlin et al. (1988) J. Viral. 62:1963-1973 "Adeno-Associated General Transduction Vectors: Analysis of Proviral Structures"
12. Lebkowski et al. (1988) Mol. Cell Biol. 8:3988-3996 "Adeno-Associated Virus: a Vector System for Efficient Introduction and Integration of DNA into a Variety of Mammalian Cell Types"
13. Samulski et al. (1989) J. Virol. 63:3822-3828 "Helper-Free Stocks of Recombinant Adeno-Associated Viruses: Normal Integration Does not Require Viral Gene Expression"
14. Srivastava et al. (October 1989) Proc. Natl. Acad. Sci. U.S.A. 86:20, 8078-82 "Construction of a recombinant human parvo virus-B19: adeno-associated virus-2 (AAV) DNA inverted terminal repeats are functional in an AAV-B19 hybrid virus -vector construction; potential application gene cloning in bone marrow cell culture and gene therapy"
15. Ohi, S. et al. (1990) J. Cell.Biochem. (Suppl.14A,D422) "Construction of recombinant adeno-associated virus that harbors human beta-globin cDNA - vector construction for potential application in hemoglobinopathy gene therapy; gene cloning and expression in 293 cell culture"
16. Ohi, S. et al. (1990) Gene 89 2:279-82 "Construction and replication of an adeno-associated virus expression vector that contains human beta-globin cDNA - plasmid PAVh-beta-GHP11 and plasmid PAVh-beta-G-psi-1 construction; potential application in gene therapy of e.g. sickle cell anemia or thalassemia"
17. Ohi, S. et al. (1990) FASEB J. 4:7, A2288) "Production and expression of recombinant adeno-associated viruses harboring human beta-globin cDNA - adeno-associated virus expression in 293 cell culture; potential gene therapy for hemoglobinopathy disease"
18. Samulski et al. (1991) Embo J. 10:3941-3950 "Targeted Integration of Adeno-associated virus AAV Into human chromosome 19"
19. Ruffing et al. (Dec. 1992) J. Virol. 66:6922-6930 "Assembly of Viruslike Particles by Recombinant Structural Proteins of Adeno-Associated Virus Type 2 in Insect Cells"
20. Sitaric et al, (1991) FASEB 5:A1550 "Production of a Helper-free Recombinant Adeno-Associated Virus That Harbors Human β-globin cDNA"
21. Walsh et al. (1991) Clin. Res. 2:325 "Gene Transfer and High-level Expression of a human γ-globin Gene Mediated by a Novel Adeno-Associated Virus Promoter" 22. Carter, B.J. (October 1992) Curr. Opinion in Biotechnol. 3:533-539 "Adeno-Associated Virus Vectors"
23. Ohi et al. (1992) (June 21-22, 1991) EXP Hematol 20 119 "Synthesis of a human beta globin in the recombinant adeno-associated virus-infected cells towards gene therapy of hemoglobinopathies"
24. Flotte et al. (1993) J. B. C. 268:3781-3790 "Expression of the Cystic Fibrosis Transmembrane Conductance Regulator from a Novel Adeno-Associated Virus Promoter"
25. Wong et al. (1993) Blood 82:302A. "High efficiency gene transfer into growth arrested cells utilizing an adeno-associated virus (AAV)-based vector"
26. Shaughnessey, et al. (1994) Proc. Am. Assoc. Cancer Res. 35:373 "Adeno-associated virus vectors for MDR1 gene therapy - multidrug-resistance gene cloning and gene transfer into hematopoietic stem cell culture using adeno-associated virus vector CWRSP for potential gene therapy"
27. Tenenbaum, L. et al. (1994) Gene Ther. (1, Suppl.1,S80) "Adeno-Associated Virus (AAV) as a Vector for Gene Transfer into Glial Cells of the Human Central Nervous System - Potential Gene Therapy"
28. Friedmann, T. (1994) Gene Ther. (1, Suppl.1, S47-S48) "Gene Therapy for Disorders of the CNS - Parkinson Disease Alzheimer Disease Therapy by Gene Transfer Using Herpes Simplex Virus, Adeno Virus and Adeno-Associated Virus Vector"
29. DE 42 19626 A1 Assignee: Wehling, P. Filed: 16 June 1992
   Publication: 23 DEC 93
   "Methods for Introducing Therapeutically Relevant Genes into Cells"
30. WO 91/18088
   Assignee: Nat. Inst. Health-Bethesda
   Filed: 17 May 1991 (Priority 23 May 1990)
   Inventors: Chatterjee and Wong
   Publication: 28 November 1991
   "Adeno-Associated Virus (AAV)-based Eukaryotic Vectors"
31. EP 0 592 836 A1
   Assignee: American Cyanamide Co.
   Filed: 16 September 93 (priority 17 Sept 92 US 947127)
   Publication: 20 April 94
   "Human Adeno-Associated Virus Integration Site DNA and use thereof"
32. WO 93/24641
   Assignee: U.S. Dept. Health-Human-Serv.
   Filed: 2 June 1993 (Priority 2 June 1992)
   Publication: 20 APR 94
   "Adeno-Associated Virus with Inverted Terminal Repeat Sequences as Promoter"
33. WO 93/09239
   Assignee: Res. Corp. Technol.
   Filed: 6 NOV 92 (US priority 8 NOV 91)
   Publication: 13 MAY 93
   "Adeno-Associated Virus-2 Basal Vectors"
34. EP 0 488 528 A1
   Assignee: Appl. Immune Sci.
   Filed: 29 OCT 91 (US priority 30 OCT 90)
   Publication: 3 JUNE 92
   "Recombinant adeno-associated Virus Vectors"
35. USPN 4,797, 368
   Assignee: U.S. Dept. Health-Human-Serv.
   Filed: 15 MAR 85
   Issued: 10 JAN 89
   "Adeno-associated Virus as Eukaryotic Expression Vector"
   Two recent review article provide a particularly complete overview of the recent status of gene therapy using AAV virus and include a collection of additional recent scientific publications in this field.
36. Samulski, R. J. "Adeno-associated Viral Vectors" Chapter 3 in "Viruses in Human Gene Therapy" Chapman & Hall, J.-M. H. Vos., ed. 1994
37. Samulski, R. J. "Adeno-associated Virus-based Vectors for Human Gene Therapy" Chapter 11 in "Gene Therapy: From Laboratory to the Clinic" World Scientific, K. M. Hui, ed. 1994

Actual delivery is accomplished by using any physical method that will transport the AAV recombinant vector into the muscle tissue of a host animal. In this discussion on administration, "AAV vector" means both a bare recombinant vector and vector DNA packaged into viral coat proteins, as is well known for AAV administration. Simply dissolving an AAV vector in phosphate buffered saline has been demonstrated to be sufficient to provide a vehicle useful for muscle tissue expression, and there are no known restrictions on the carriers or other components that can be coadministered with the vector (although compositions that degrade DNA should be avoided in the normal manner with vectors). Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The vectors can be used with any pharmaceutically acceptable carrier for ease of administration and handling.

For purposes of intramuscular injection, solutions in an adjuvant such as sesame or peanut oil or in aqueous propylene glycol can be employed, as well as sterile aqueous solutions. Such aqueous solutions can be buffered, if desired, and the liquid diluent first rendered isotonic with saline or glucose. Solutions of the AAV vector as a free acid (DNA contains acidic phosphate groups) or a pharmacologically acceptable salt can be prepared in water suitably mixed with a surfactant such as hydroxypropylcellulose. A dispersion of AAV viral particles can also be prepared in glycerol, liquid polyethylene glycols and mixtures thereof and in oils. Under ordinary conditions of storage and use, these preparations contain a preservative to prevent the growth of microorganisms. In this connection, the sterile aqueous media employed are all readily obtainable by standard techniques well-known to those skilled in the art.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating the AAV vector in the required amount in the appropriate solvent with various of the other ingredients enumerated above, as required, followed by filtered sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique which yield a powder of the active ingredient plus any additional desired ingredient from the previously sterile-filtered solution thereof.

For purposes of topical administration, dilute sterile, aqueous solutions (usually in about 0.1% to 5% concentration), otherwise similar to the above parenteral solutions, are prepared in containers suitable for incorporation into a transdermal patch, and can include known carriers, such as pharmaceutical grade dimethylysulfoxide (DMSO).

The therapeutic compounds of this invention may be administered to a mammal alone or in combination with pharmaceutically acceptable carriers. As noted above, the relative proportions of active ingredient and carrier are determined by the solubility and chemical nature of the compound, chosen route of administration and standard pharmaceutical practice.

The dosage of the present therapeutic agents which will be most suitable for prophylaxis or treatment will vary with the form of administration, the particular compound chosen and the physiological characteristics of the particular patient under treatment. Generally, small dosages will be used initially and, if necessary, will be increased by small increments until the optimum effect under the circumstances is reached. Exemplary dosages are set out in the example below.

Since AAV has in the past been shown to have a broad host range (for pulmonary expression) and has now been demonstrated to be operable in the muscle tissue, there are no known limits on the animals in which muscle tissue expression can take place, particularly in mammals, birds, fish, and reptiles, especially domesticated mammals and birds such as cattle, sheep, pigs, horses, dogs, cats, chickens, and turkeys. Both human and veterinary uses are particularly preferred.

The gene being expressed can be either a DNA segment encoding a protein, with whatever control elements (e.g., promoters, operators) are desired by the user, or a non-coding DNA segment, the transcription of which produces all or part of some RNA-containing molecule (such as a transcription control element, +RNA, or anti-sense molecule). Since the present invention is directed to a route of delivery and to the vector rather than to the material being delivered, there are no limitations on the foreign DNA (non-AAV DNA) being delivered by the vector. The gene need not be limited to those strictly useful in muscle, since the ability of the host's vascular system to deliver the gene product to other parts of the host's body will be readily apparent.

Muscle tissue is a very attractive target for *in vivo* gene delivery and gene therapy, because it is not a vital organ and is very easy to access. If a disease is caused by a defective gene product which is required to be produced and/or secreted, such as hemophilia, diabetes and Gaucher's disease, etc., muscle will be a good candidate to supply the gene product if the appropriate gene can be effectively delivered into the cells.

Different vectors, such as naked DNA, adenovirus and retrovirus, have been utilized to directly deliver various transgenes into muscle tissues. However, neither system can offer both high efficiency and long term expression. For naked plasmid DNA directly delivered into muscle tissue, the efficiency is not high. There are only a few cells near the injection site that can maintain transgene expression. Furthermore, the plasmid DNA in the cells remains as non-replicating episomes, i.e. in the unintegrated form. Therefore, it will be eventually lost. For adenovirus vector, it can infect the non-dividing cells, and therefore, can be directly delivered into the mature tissues such as muscle. However, the transgene delivered by adenovirus vectors are not useful to maintain long term expression for the following reasons. First, since adenovirus vectors still retain most of the viral genes, they are not very safe. Moreover, the expression of those genes can cause the immune system to destroy the cells containing the vectors (see, for example, Yang et al. 1994, Proc. Natl Acad. Sci. 91:4407-4411). Second, since adenovirus is not an integration virus, its DNA will eventually be diluted or degraded in the cells. Third, due to the immune response, adenovirus vector could not be repeatedly delivered. In the case of lifetime diseases, this will be a major limitation. For retrovirus vectors, although they can achieve stable integration into the host chromosomes, their use is very restricted because they can only infect dividing cells while a large majority of the muscle cells are non-dividing.

Adeno-associated virus vectors have certain advantages over the above-mentioned vector systems. First, like adenovirus, AAV can efficiently infect non-dividing cells. Second, all the AAV viral genes are eliminated in the vector. Since the viral-gene-expression-induced immune reaction is no longer a concern, AAV vectors are safer than Ad vectors. Third, AAV is an integration virus by nature, and integration into the host chromosome will stably maintain its transgene in the cells. Fourth, AAV integrates into a specific region of human chromosome 19. Therefore, it has a safety advantage over retroviruses, which insert more randomly into the host chromosome. Fifth, AAV is an extremely stable virus, which is resistant to many detergents, pH changes and heat (stable at 56° C for more than an hour). It can also be lyophilized and redissolved without losing its activity. Therefore, it is a very promising delivery vehicle for gene therapy.

The inventors have demonstrated the principle of the invention using AAV vectors containing a LacZ reporter gene as a model system to explore the potential application of AAV vector in muscle tissue by directly injecting the vector into the leg muscles of mice. At the same time, we have compared an adenovirus vector, Ad-LacZ, with the AAV-LacZ vector in the *in vivo* experiments.

### EXAMPLE

### Preparation of AAV viral vector

AAV-LacZ viral particles were produced by cotransfecting the vector plasmid pAB-11 with the helper plasmid pAAV/Ad into adenovirus infected 293 cells (Samulski et al. J. Virol. 63:3822 1989). pAB11 was prepared as described in Goodman et al. Blood 1994 84:1492-1500. Briefly, 25 µg of plasmid DNA (6 µg vector plus 19 µg helper) was transfected by calcium phosphate precipitation into 239 cells at 80% confluency in Dulbecco's Modified Eagle Medium (DMEM) plus 10% fetal calf serum (FCS). The medium was replaced after 8 to 12 hour transfection with fresh DMEM plus 2 % FCS. Adenovirus 5 was added to the cells at 1 m.o.i. (multiplicity of infection). After two and one-half days, the cells were harvested and then frozen and thawed three times. Cell debris was removed by low speed centrifugation.

The supernatant containing AAV-LacZ was gently extracted 2 to 3 times with an equal volume of chloroform. The residue chloroform was eliminated by nitrogen gas blowing. To the supernatant, one-third volume of saturated ammonium sulfate solution was added to make 25% saturation. The sample was placed on ice for 10 minutes and centrifuged at 10,000g for 10 minutes. The supernatant was recovered and saturated ammonium sulfate solution was added to make 50% saturation. The sample was then placed on ice for 10 minutes and centrifuged at 15,000g for 10 minutes. The pellet was redissolved in CsCl-PBS solution (density 1.38g/ml) and centrifuged at 40,000 rpm in a SW41 rotor (Beckman) for 48 hours. The AAV band was collected, dialyzed against DMEM and heated at 56° C for 15 to 30 minutes. The AAV-LacZ virus titers were determined by infecting 293 cells at various dilutions. The cells were fixed and stained with X-gal (Dhawan et al. 1991 Science 254:1509-1512).

The Ad-LacZ vector was prepared as described in Yang et al. (J. Virol. 1995, 69:2004-2015; Proc. Natl Acad. Sci, 1994, 91:4407-4411) and the references therein.

### Injection of AAV viral vector into the muscle tissue

In detail, 3-week-old mice from two litters were randomly divided into two groups. Before the injection, the animals were anesthetized i.p. with 0.018 ml of 2.5% Avertin per gram of body weight. In the first group, 30 µl of AAV-LacZ (3x10⁶ infectious units) was injected into the left hind leg and 30 µl of Ad-LacZ (3x10⁶ infectious units) into the right leg. In the second group, 30 µl of AAV-LacZ (3x10⁶ units) was injected into the left leg and 30 µl mix of AAV-LacZ plus Ad-LacZ (3x10⁶ infectious units each) was injected into the right leg. The AAV-LacZ encoded β-galactosidase contains a nuclear localization signal while the Ad-LacZ encoded β-galactosidase is cytoplasmic. Therefore, the gene expression in the muscle cells from the two vectors can be distinguished.

### Detection of transgene expression of the vectors in the muscle

At various time points, the mice were sacrificed and muscle tissue was harvested. The samples were quickly frozen in the liquid nitrogen and 20µm cryo thin sectioning was performed. The sections were then fixed, washed with PBS, and stained with X-gal solution overnight.

### Analysis of Results

After injection of 30 µl (3X10⁶ infectious units) of AAV-LacZ and/or Ad-LacZ virus, the mice were sacrificed at different time points and the tissues were stained for LacZ expression. The AAV-LacZ and Ad-LacZ started to express their transgene as early as 48 hours after virus delivery (data not shown). Strong immune response as lymphocyte infiltration was observed in the Ad-LacZ and Ad-LacZ + AAV-LacZ injection sites, whereas much less reaction was seen in AAV-LacZ alone injection site. At the three-week time point, the lymphocyte infiltration mostly disappeared. At this point, however, only a few cells remained positive for X-gal staining at the Ad-LacZ injected site. Nevertheless, hundreds of muscle myotubes remained positive for X-gal staining at either the AAV-LacZ alone site or at the AAV-LacZ + Ad-LacZ site. The distinctive nuclear staining indicates that the LacZ transgene expression in those cells was from AAV-LacZ vector instead of Ad-LacZ vector. These results demonstrated that AAV vector can efficiently deliver transgene into muscle cells and that the Ad-LacZ can cause stronger immune reaction than the AAV-LacZ does. This appears to result from the adenovirus vector containing not only the transgene but also numerous viral genes while AAV vector only possesses the transgene. The likely viral gene expression appears to induce a strong immune response from the host that will eventually eliminate the adenovirus-transduced cells but not the AAV-transduced cells.

### AAV vector can have long term transgene expression

From 4 days up to 5 months, no obvious decrease of LacZ gene expression from AAV-LacZ vector was observed. However, for Ad-LacZ, almost no LacZ staining was visible after three weeks. In the adenovirus-injected sites, the cytoplasmic LacZ staining disappeared along with the disappearance of lymphocyte infiltration. However, in AAV-LacZ samples, the nuclear LacZ staining persisted while the infiltration fully (or nearly so) disappeared (occasionally a few lymphocytes could be seen around some of the blue cells.

The above results lead to the following conclusions:
First, AAV vector can efficiently deliver transgene into the mouse muscle tissue. At the concentration used here, the AAV vector is more efficient than the Ad vector.
Second, AAV transduction into muscle cells does not need cell division. This is supported by the high percentage transduction (close to 100% in certain areas) of the muscle cells, since most of them are non-dividing at three weeks of age when the viruses were injected.
Third. AAV vector can offer long term transgene expression in muscle cells, up to 5 months, indicating that the promoter used in AAV vector was not shut off and that the AAV transduced cells were not eliminated by the host immune system.

Finally, we have demonstrated that AAV can be used as an efficient, safe and practical gene therapy vector, by directly injecting the target gene embodied in AAV vector into muscle tissues. As a result, many metabolic diseases such as Gaucher's disease, endocrine diseases such as diabetes, and coagulation diseases such as hemophilia. A and B as well as certain muscular diseases, will be suitable candidates for AAV vector mediated gene therapy.

All publications and patent applications mentioned in this specification are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

## Claims

1. Use of a recombinant AAV vector comprising a non-AAV gene capable of expressing a gene product, ligated into an AAV vector, for the preparation of a pharmaceutical composition for the treatment of a disease caused by a deficiency of said gene product, which is required to be produced and/or secreted in an animal, wherein the composition is administered into muscle tissue of an animal.

2. The use of claim 1, wherein said composition comprising said vector is administered dissolved or suspended in a liquid pharmaceutically acceptable carrier.

3. The use of claim 2, wherein said liquid carrier comprises an aqueous solution.

4. The use of any preceding claim, wherein said gene comprises a DNA segment encoding a protein operably linked to a promoter operable in said muscle tissue.

5. The use of any preceding claim, wherein said administering is by intramuscular injection.

6. The use of any of claims 1 to 4, wherein said administering is by transdermal transport.

7. The use of any preceding claim, wherein said AAV vector comprises non-AAV DNA ligated into an AAV genome in place of or in addition to an AAV DNA sequence excluding the first and last 145 basepairs of said AAV genome or non-AAV DNA operably linked to a vector comprising a double-D AAV genomic segment consisting of 165 basepairs including an internal terminal repeat with D segments at both ends.

8. The use of any preceding claim, wherein said gene comprises a DNA segment which is capable of being transcribed to produce an RNA molecule encoding a protein and having translational start and stop signals for said protein.

9. The use of any preceding claim, wherein said animal is a bird or mammal.

10. The use of any preceding claim, wherein said animal is a human.

11. The use of any preceding claim, wherein said non-AAV gene of interest encodes, β-galactosidase.

12. The use of any of claims 1 to 10, wherein the disease is a coagulation disease such as hemophilia, an endocrine disease such as diabetes or a metabolic disease such as Gaucher's disease.

## Patentansprüche

1. Verwendung eines rekombinanten AAV-Vektors, umfassend ein Nicht-AAV-Gen, das in der Lage ist, ein Genprodukt zu exprimieren und in einen AAV-Vektor ligiert ist, für die Herstellung einer pharmazeutischen Zusammensetzung für die Behandlung einer Krankheit, die durch eine Defizienz des Genprodukts verursacht ist, dessen Herstellung und/oder Sezernierung in einem Tier erforderlich ist, wobei die Zusammensetzung in das Muskelgewebe eines Tieres verabreicht wird.

2. Verwendung nach Anspruch 1, wobei die Zusammensetzung, die den Vektor umfasst, in einem flüssigen pharmazeutisch verträglichen Träger aufgelöst oder suspendiert verabreicht wird.

3. Verwendung nach Anspruch 2, wobei der flüssige Träger eine wässrige Lösung umfasst.

4. Verwendung nach einem der vorangegangenen Ansprüche, wobei das Gen ein DNA-Segment umfasst, das ein Protein kodiert und funktionell mit einem Promotor verbunden ist, der im Muskelgewebe funktionsfähig ist.

5. Verwendung nach einem der vorangegangenen Ansprüche, wobei die Verabreichung durch intramuskuläre Injektion erfolgt.

6. Verwendung nach einem der Ansprüche 1 bis 4, wobei die Verabreichung durch transdermalen Transport erfolgt.

7. Verwendung nach einem der vorangegangenen Ansprüche, wobei der AAV-Vektor Nicht-AAV-DNA umfasst, die in ein AAV-Genom ligiert ist, und zwar anstelle oder zusätzlich zu einer AAV-DNA-Sequenz, die die ersten und letzten 145 Basenpaare des AAV-Genoms nicht einschließt, oder Nicht-AAV-DNA umfasst, die mit einem Vektor funktionell verbunden ist, wobei der Vektor ein doppel-D AAV genomisches Segment umfasst, das aus 165 Basenpaaren besteht und einen internen terminalen Repeat mit D-Segmenten an beiden Enden einschließt.

8. Verwendung nach einem der vorangegangenen Ansprüche, wobei das Gen ein DNA-Segment umfasst, das transkribiert werden kann, sodass ein RNA-Molekül hergestellt wird, das ein Protein kodiert und Translationsstart- und -stoppsignale für das Protein aufweist.

9. Verwendung nach einem der vorangegangenen Ansprüche, wobei das Tier ein Vogel oder ein Säugetier ist.

10. Verwendung nach einem der vorangegangenen Ansprüche, wobei das Tier ein Mensch ist.

11. Verwendung nach einem der vorangegangenen Ansprüche, wobei das Nicht-AAV-Gen von Interesse β-Galaktosidase kodiert.

12. Verwendung nach einem der Ansprüche 1 bis 10, wobei die Krankheit eine Koagulationskrankheit wie Hämophilie, eine endokrine Krankheit wie Diabetes oder eine metabolische Krankheit wie Gaucher-Krankheit ist.

## Revendications

1. Utilisation d'un vecteur AAV recombinant comprenant un gène non AAV capable d'exprimer un produit de gène, ligaturé dans un vecteur AAV, pour la préparation d'une composition pharmaceutique pour le traitement d'une maladie causée par une déficience dudit produit de gène, qui est requis pour être produit et/ou sécrété chez un animal, dans laquelle la composition est administrée dans le tissu musculaire d'un animal.

2. Utilisation selon la revendication 1, dans laquelle ladite composition comprenant ledit vecteur est administrée dissoute ou en suspension dans un support liquide pharmaceutiquement acceptable.

3. Utilisation selon la revendication 2, dans laquelle ledit support liquide comprend une solution aqueuse.

4. Utilisation selon l'une des revendications précédentes, dans laquelle ledit gène comprend un segment d'ADN codant une protéine liée de façon opérationnelle à un promoteur opérationnel dans ledit tissu musculaire.

5. Utilisation selon l'une des revendications précédentes, dans laquelle ladite administration est réalisée par injection intramusculaire.

6. Utilisation selon l'une des revendications 1 à 4, dans laquelle ladite administration est réalisée par transport transdermique.

7. Utilisation selon l'une des revendications précédentes, dans laquelle ledit vecteur AAV comprend de l'ADN non AAV ligaturé dans un génome AAV à la place de ou en plus d'une séquence d'ADN d'AAV excluant les 145 premières et dernières paires de bases dudit génome AAV ou ADN non AAV lié de façon opérationnelle à un vecteur comprenant un segment génomique d'AAV double D consistant en 165 paires de bases incluant une répétition terminale interne avec des segments D aux deux extrémités.

8. Utilisation selon l'une des revendications précédentes, dans laquelle ledit gène comprend un segment d'ADN qui est capable d'être transcrit pour produire une molécule d'ARN codant une protéine et ayant des signaux d'initiation et de terminaison de traduction pour ladite protéine.

9. Utilisation selon l'une des revendications précédentes, dans laquelle ledit animal est un oiseau ou un mammifère.

10. Utilisation selon l'une des revendications précédentes, dans laquelle ledit animal est un humain.

11. Utilisation selon l'une des revendications précédentes, dans laquelle ledit gène non AAV d'intérêt code pour la β-galactosidase.

12. Utilisation selon l'une des revendications 1 à 10, dans laquelle la maladie est un trouble de la coagulation telle que l'hémophilie, une maladie endocrine telle que le diabète ou une maladie métabolique telle que la maladie de Gaucher.
